# FASCICULE DE BREVET EUROPEEN

(11) **EP 0 599 728 B1**
(45) Date de publication et mention de la délivrance du brevet: **23.07.1997**
(21) Numéro de dépôt: 93402840.8
(22) Date de dépôt: 22.11.1993
(51) Int. Cl.: C07C 13/19, C07C 5/05

(54) **Procédé pour l'hydrogénation sélective de composés comportant des insaturations endo et exocycliques**
Verfahren zur selektiven Hydrierung von Verbindungen mit endo- und exocyklischen ungesältigten Bindungen
Process for the selective hydrogenation of compounds containing endo- and exocyclic insaturations

(30) Priorité: 26.11.1992 FR 9214353
(43) Date de publication de la demande: 01.06.1994
(73) Titulaire: INSTITUT FRANCAIS DU PETROLE, 92502 Rueil-Malmaison (FR)
(72) Inventeur: Lucas, Christine, F-69330 Jonage (FR); Basset, Jean-Marie, F-69100 Villeurbane (FR); Boitiaux, Jean-Paul, F-78300 Poissy (FR); Candy, Jean-Pierre, F-69300 Caluire (FR); Didillon, Blaise, F-92500 Rueil Malmaison (FR)
(74) Mandataire: Andreeff, François

(56) Documents cités:
- DD-A- 106 343

## Description

L'invention concerne un procédé d'hydrogénation d'un composé cyclique polyinsaturé, comportant au moins une double liaison carbone-carbone endocyclique (c'est-à-dire dans le cycle) et au moins une double liaison carbone-carbone exocyclique (hors du cycle) terminale, l'hydrogénation se faisant sélectivement sur la(les) double(s) liaison(s) endocyclique(s).

Le procédé nécessite l'emploi d'un catalyseur à base d'un métal du groupe VIII et d'un métal additionnel appartenant au groupe IVa, famille de l'étain (germanium, étain, plomb).

L'invention s'applique particulièrement bien à l'hydrogénation catalytique du 4-vinylcyclohexène en vinylcyclohexane.

Le 4-vinylcyclohexène (I) est un composé contenant deux doubles liaisons carbone-carbone, l'une endocyclique et l'autre exocyclique.

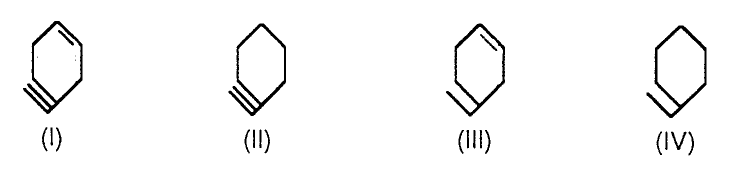

L'hydrogénation catalytique du 4-vinylcyclohexène (I) peut conduire d'abord au vinylcyclohexane (II) ou à l'éthyl-cyclohexène (III). Ces deux produits peuvent ensuite être hydrogénés en éthyl-cyclohexane (IV).

La réduction catalytique du 4-vinylcyclohexène (I) a fait l'objet d'un certain nombre de travaux que ce soit sur des catalyseurs hétérogènes (US 4 716 256 par exemple) ou homogènes. Mais dans la plupart des cas rapportés dans la littérature les deux produits majoritaires obtenus sont l'éthyl-cyclohexène (III) et le l'éthyl-cyclohexane (IV).

Au contraire, dans le brevet US 3,154,594 le vinylcyclohexane est préparé à partir du 4-vinylcyclohexène en faisant réagir le 4-vinylcyclohexène avec un organoaluminium (triisobutyl aluminium par exemple), le composé obtenu est hydrogéné en présence d'un catalyseur choisi dans le groupe formé par le palladium, le platine, le nickel Raney. Par chauffage du produit hydrogéné à 100-200 °C en présence d'une oléfine ayant une insaturation terminale (dodécène-1 par exemple), le vinylcyclohexane est isolé.

Le document DD-106.343 décrit un procédé d'hydrogénation sélective des liaisons endocycliques du 2-vinylbicyclo-[2,2,1]-heptène avec un catalyseur au Palladium, et en particulier un catalyseur constitué de 0,5% Pd et d'alumine gamma chargés avec 15% CaO.

Il a été découvert dans la présente invention qu'il est possible de réaliser en une seule étape l'hydrogénation sélective du 4-vinyl-cyclohexène (I) en vinyl-cyclohexane (II) sans obtention d'éthyl-cyclohexène et en limitant la production d'éthyl-cyclohexane (IV). On opère dans un réacteur continu ou discontinu en présence d'hydrogène sous une pression totale comprise entre 1 et 10 MPa et de préférence entre 1 et 5 MPa et à une température comprise entre 0 et 100 °C et de préférence entre 20 et 50°C en présence d'un catalyseur renfermant un support et (a) au moins un métal du groupe VIII choisi dans le groupe constitué par l'iridium, l'osmium, le nickel, le palladium, le platine, le rhodium (le rhodium étant le métal préféré) et dont le pourcentage pondéral est choisi entre 0,1 et 5 % et de préférence entre 1 et 3 %. et (b) au moins un métal additionnel choisi dans le groupe IVa constitué par l'étain, le germanium et le plomb dont le pourcentage pondéral est choisi entre 0,01 % et 15 %. Le rapport molaire élément du groupe VIII, élément du groupe IVa est compris entre 0,3 et 3 et de préférence entre 0,8 et 2,5. Le support peut être choisi parmi les oxydes réfractaires dans le groupe constitué par la silice, l'alumine, les silices alumines ainsi que les supports à base de carbone et notamment le charbon et le graphite.

Le catalyseur peut être préparé par différentes procédures d'imprégnation du support. L'opération d'imprégnation consiste par exemple à mettre en contact le support avec une solution aqueuse ou organique d'un composé du métal ou des métaux choisis. On peut introduire le (ou les) métal(aux) du groupe VIII et le (ou les) métal(aux) additionnel(s) simultanément ou successivement. Après avoir laissé le contact entre le support et la solution pendant plusieurs heures le support imprégné est filtré, lavé à l'eau ou par un hydrocarbure, séché et calciné sous air entre 110°C et 600°C et préférentiellement entre 100°C et 500°C.

De préférence, on opère avec deux imprégnations successives : le support subit d'abord une imprégnation, à l'aide d'une solution aqueuse ou organique d'au moins un composé de métal du groupe VIII. Le support imprégné est ensuite filtré, séché éventuellement lavé à l'eau ou par un solvant organique et calciné sous air habituellement entre 110°C et 600°C et de préférence entre 110°C et 500°C puis réduit sous hydrogène à une température comprise entre 200°C et 600°C environ et de préférence entre environ 300°C et 500°C; le produit obtenu est alors imprégné par une solution aqueuse ou organique d'un composé du germanium, de l'étain et/ou du plomb. De manière avantageuse on utilise une solution d'au moins un alkyl ou aryl germanium, alkyl ou aryl étain, alkyl ou aryl plomb selon la technologie décrite dans le brevet US-A-4.548.918. de la demanderesse.

Parmi les solvants organiques utilisables dans le cadre de l'invention on peut citer à titre d'exemple les hydrocarbures, les hydrocarbures halogénés, les cétones, les éthers et les dérivés aromatiques. Lorsque le composé du germanium de l'étain ou du plomb est liquide ou gazeux dans les conditions d'imprégnation, le solvant n'est pas indispensable.

Après avoir laissé le contact entre le support imprégné du ou des métaux du groupe VIII et la solution contenant au moins un composé du groupe IVa pris parmi le germanium, l'étain ou le plomb pendant un temps déterminé compris entre 0,2 et 10 heures à une température généralement comprise entre 20°C et 150°C, le produit est éventuellement lavé à l'aide du solvant utilisé pour imprégner le composé du groupe IVa, éventuellement séché et éventuellement calciné sous air à une température comprise entre 90°C et 600°C et réduit entre 50 et 600°C ou éventuellement utilisé directement après l'imprégnation du ou des composés du groupe IVa.

Une autre méthode consiste à malaxer la poudre humide de support avec les précurseurs du catalyseur et à mettre ensuite en forme et à sécher.

Les exemples des précurseurs métalliques utilisables dans la préparation du catalyseur sont les suivants: pour les métaux du groupe VIII on peut utiliser des composés tels que les chlorures, les nitrates, les composés halogénoaminés, les composés aminés, les sels d'acides organiques.

On peut aussi utiliser des composés organométalliques d'un métal du groupe VIII en solution dans un solvant organique, par exemple un hydrocarbure. Comme exemples d'hydrocarbures ont peut citer les hydrocarbures paraffiniques saturés dont la chaîne renferme de 6 à 12 atomes de carbone par molécule ou encore les hydrocarbures aromatiques renfermant un nombre de carbone équivalent. A titre d'exemples de composés organométalliques de métal du groupe VIII on peut citer les composés carbonyles, halogénocarbonyles, les acétylacétonates sans que cette liste soit limitative.

L'élément choisi dans le groupe constitué par l'étain le germanium et le plomb peut être introduit sous la forme de complexes polycétoniques ou hydrocarbyles tels que les alkyles, les aryles, les alkylaryles. L'introduction du ou des métaux du groupe IVa est avantageusement effectuée à l'aide d'une solution dans un solvant organique du complexe organométallique du dit métal. Comme complexe organométallique du métal du groupe IVa on citera en particulier le tétrabutylétain, le tétraméthylétain, le tétraéthylétain, le tétrapropylétain, le tétraéthylgermanium, le tétraméthylgermanium, le tétraéthylplomb, le diphénylétain, l'hydrure de tributylétain, le chlorure de tributylétain, cette liste n'étant pas exhaustive. Le solvant d'imprégnation est choisi dans le groupe constitué par les hydrocarbures paraffiniques, naphténiques, aromatiques contenant de 6 à 12 atomes de carbone par molécule et les composés organiques halogénés contenant 1 à 12 atomes de carbone par molécule. On peut citer le n-heptane, le méthylcyclohexane, le toluène, le chloroforme. On peut aussi utiliser des mélanges bien définis des solvants ci-dessus ou d'autres solvants.

L'élément choisi dans le groupe constitué de l'étain, du germanium ou du plomb peut aussi être introduit par l'intermédiaire de composés tels que les chlorures, les bromures, les nitrates, les acétates d'étain; les oxydes, les oxalates les chlorures de germanium; les halogénures, les nitrates, l'acétate de plomb en solution aqueuse ou organique.

Le support peut être de nature variée comme déjà mentionné plus haut. Un support particulièrement adapté possède des caractéristiques spécifiques telles qu'une aire spécifique déterminée par la méthode BET comprise entre 10 et 500 m² par gramme et un volume poreux total de 0,2 à 1,3 cm³ par gramme de support.

Une fois les métaux fixés sur le support, le catalyseur peut éventuellement subir un traitement d'activation sous hydrogène entre 50 et 600°C. Cette étape n'est cependant pas toujours nécessaire.

Les exemples suivants, non limitatifs, illustrent l'invention.

### Exemple 1 (Essai comparatif)

La préparation du catalyseur se fait par imprégnation de chlorure de rhodium chloropentamine en solution ammoniacale sur une silice dont la surface spécifique est égale à 200 m² par gramme et le volume poreux total à 0,8 cm³ par gramme, suivie d'une filtration, d'un lavage à l'eau distillé, d'une calcination sous air à 450°C.

Le catalyseur fini contient 1,9 % de rhodium et sera appelé le catalyseur A.

Le catalyseur A (0,0075 g) est ensuite mélangé avec 0,1425 g de silice pour conduire au catalyseur B. Le catalyseur B est ensuite réduit sous hydrogène à 450 °C chargé sous courant d'argon dans le réacteur de type grignard contenant un solvant organique (n-heptane). Le réacteur est ensuite fermé, purgé de l'argon contenu. Sous excès d'hydrogène, on injecte alors une solution de 4-vinyl-cyclohexène (I) correspondant à une concentration initiale de 0,154 mol/l de 4-vinyl-cyclohexène (I). La pression d'hydrogène est ensuite augmentée jusqu'à 5 MPa; la température est maintenue constante à 22°C. L'évolution de la composition du milieu réactionnel est suivie par chromatographie en phase gazeuse.

Les résultats obtenus sont rapportés dans le tableau 1.

**Tableau 1**

| **Temps minutes** | **Conversion (%)** | **Sélectivité en (%)** | |
|---|---|---|---|
| | | **(II)** | **(IV)** |
| 0 | 0 | - | - |
| 5 | 85 | 5,5 | 87 |
| 10 | 100 | 0 | 98 |

On voit que l'activité du catalyseur B est élevée. Par contre, les sélectivités en 4-vinyl-cyclohexane (II) sont faibles. Le catalyseur B conduit majoritairement à la réduction des deux insaturations du 4-vinylcyclohexène avec formation d'éthyl-cyclohexane (IV).

### Exemple 2 (selon l'invention)

A partir du catalyseur A un catalyseur C rhodium-étain est préparé selon le protocole suivant. Le catalyseur A est d'abord réduit sous hydrogène à 450°C. La fixation de l'étain se fait phase gazeuse: le tétrabutylétain est injecté directement sur le catalyseur A à température ambiante sous 2 kPa d'hydrogène, puis la température est augmentée de façon contrôlée, à une vitesse de 100°C/h jusqu'à 350°C tout en maintenant une faible pression d'hydrogène. Le catalyseur C ainsi obtenu contient 1,9 % poids de rhodium et 2 % poids d'étain.

Le catalyseur C est testé en hydrogénation du vinylcyclohexène dans les conditions de l'exemple 1 (catalyseur B).

Les résultats obtenus sont rapportés dans le tableau 2.

**Tableau 2**

| **temps minutes** | **conversion (%)** | **sélectivité en %** | |
|---|---|---|---|
| | | **(II)** | **(IV)** |
| 0 | 0 | - | - |
| 15 | 31 | 91 | 8 |
| 60 | 62 | 85 | 15 |
| 350 | 100 | 0 | 100 |

Bien que le catalyseur C soit moins actif que le catalyseur B, il permet d'obtenir des sélectivités en vinyl-cyclohexane (II) nettement meilleures puisqu'à des conversions inférieures à 60 % la sélectivité en vinylcyclohexane (II) est supérieure à 85 %.

### Exemple 3 (selon l'invention)

A partir du catalyseur A un catalyseur D rhodium-étain est préparé selon le protocole déjà décrit dans la littérature (US 4,456,775). L'étain est imprégné sous forme de tétrabutylétain en solution dans le normal heptane. Après avoir laissé en contact pendant 1 heures le catalyseur et la solution de tétrabutylétain à la température de reflux du n-heptane, le catalyseur est lavé par du n-heptane puis séché sous atmosphère d'azote. Le catalyseur D ainsi obtenu contient 1,9 % poids de rhodium et 2 % poids d'étain.

Le catalyseur D (0,150 g) est ensuite chargé sous courant d'argon dans le réacteur de type grignard contenant un solvant organique (n-heptane). Le réacteur est ensuite fermé, purgé de l'argon contenu. Sous hydrogène, on injecte alors une solution de 4-vinyl-cyclohexène (I) correspondant à une concentration initiale de 0,154 mol/l de 4-vinyl-cyclohexène (I)

L'hydrogénation du 4-vinyl-cyclohexène (I) est alors réalisée dans les conditions de l'exemple 1.

**Tableau 3**

| **temps minutes** | **conversion (%)** | **sélectivité en (%)** | |
|---|---|---|---|
| | | **(II)** | **(IV)** |
| 0 | 0 | - | - |
| 30 | 41 | 92 | 7 |
| 60 | 63 | 88 | 12 |
| 90 | 74 | 87 | 12 |
| 120 | 82 | 85 | 15 |
| 360 | 100 | 15 | 95 |

Le catalyseur D est d'activité comparable au catalyseur C mais permet d'obtenir des rendements en vinylcyclohexane légèrement supérieurs.

### Exemple 4 (selon l'invention)

Le catalyseur C (0,150 g) préparé dans l'exemple 2 est chargé dans un réacteur de type grignard contenant un solvant organique le normal heptane avec les précautions mentionnées dans l'exemple 2. Le réacteur est ensuite fermé, purgé de l'argon contenu. Sous hydrogène, on injecte alors une solution de 4-vinyl-cyclohexène (I) correspondant à une concentration initiale de 0,154 mol/l de 4-vinyl-cyclohexène. (I) La pression d'hydrogène est ensuite augmentée jusqu'à 2 MPa; la température est maintenue constante à 22°C. L'évolution de la composition du milieu réactionnel est suivie par chromatographie en phase gazeuse.

Les résultats obtenus sont rapportés dans le tableau 4.

**Tableau 4**

| **temps minutes** | **conversion (%)** | **sélectivité en (%)** | |
|---|---|---|---|
| | | **(II)** | **(IV)** |
| 0 | 0 | - | - |
| 15 | 21 | 92 | 8 |
| 60 | 42 | 90 | 9 |
| 350 | 85 | 87 | 13 |

Sous plus faible pression d'hydrogène (2 MPa), le catalyseur C est moins actif que sous pression élevée (5 MPa) mais l'abaissement de la pression permet d'augmenter le rendement en vinyl-cyclohexène qui peut atteindre 74 %.

L'homme du métier choisira les conditions opératoires (pression, température, quantité H₂, temps de séjour, quantité de catalyseur) selon la sélectivité en produit (II) souhaité pour une conversion donnée.
L'invention s'applique particulièrement bien aux cyclohexène substitués.

## Revendications

1. Procédé pour l'hydrogénation sélective, en présence d'un catalyseur, d'un composé cyclique polyinsaturé, comportant au moins une double liaison carbone-carbone endocyclique et au moins une double liaison carbone-carbone exocyclique terminale, lesdits doubles liaisons n'étant pas conjuguées, en un mélange renfermant essentiellement le composé dont le cycle a été saturé et qui comporte au moins une double liaison exocyclique terminale, procédé dans lequel le composé cyclique polyinsaturé est mis en contact, en présence d'hydrogène sous 1 à 10 MPa et à une température de 0-100°C, avec un catalyseur renfermant un support choisi dans le groupe constitué par l'alumine, la silice, les silice-alumines et les supports à base de carbone, et
a) 0,1 à 5% en poids d'au moins un métal du groupe VIII choisi dans le groupe constitué par l'iridium, l'osmium, le nickel, le palladium, le platine, le rhodium,
et
b) 0,01 à 15% en poids d au moins un métal du groupe IVa choisi dans le groupe constitué par l'étain, le germanium et le plomb, le rapport molaire entre le(s) dit(s) métal(aux) du groupe VIII et celui(ceux) du groupe IVa étant compris entre 0,3 et 3.

2. Procédé selon la revendication 1 dans lequel le catalyseur est essentiellement constitué du support, d'au moins un métal du groupe VIII et d'au moins un métal du groupe IVa.

3. Procédé selon la revendication 1 dans lequel le composé cyclique polyinsaturé est un cyclohexène substitué.

4. Procédé selon l'une des revendications 1 ou 2, dans lequel le composé polyinsaturé est le 4-vinylcyclohexène qui est hydrogéné en vinylcyclohexane.

5. Procédé selon l'une des revendications 1 à 3, dans lequel la pression est comprise entre 1 et 5 MPa.

6. Procédé selon l'une des revendications 1 à 3, dans lequel la température est comprise entre 20 et 50°C.

7. Procédé selon l'une des revendications précédentes, dans lequel le métal du groupe VIII est le rhodium et le métal du groupe IVa l'étain.

8. Procédé selon l'une des revendications précédentes, dans lequel le catalyseur contient 1 à 3% d'au moins un métal du groupe VIII.

9. Procédé selon l'une des revendications précédentes, dans lequel le rapport molaire entre métal(aux) du groupe VIII et métal(métaux) du groupe IVa est compris entre 0,8 et 2,5.

## Claims

1. A process for the selective hydrogenation, in the presence of a catalyst, of a polyunsaturated cyclic compound comprising at least one endocyclic carbon-carbon double bond and at least one terminal exocyclic carbon-carbon double bond, said double bonds not being conjugated, to form a mixture essentially containing the compound whose ring was saturated and which comprises at least one terminal exocyclic double bond, in which process the polyunsaturated cyclic compound is brought into contact in the presence of hydrogen at from 1 to 10 MPa and at a temperature of from 0 to 100°C with a catalyst including a support selected from the group formed by alumina, silica, silica-alumina and the supports based on carbon and
a) from 0.1 to 5% by weight of at least one metal from group VIII selected from the group formed by iridium, osmium, nickel, palladium, platinum and rhodium, and
b) from 0.01 to 15% by weight of at least one metal from group IVa selected from the group formed by tin, germanium and lead, the molar ratio between said metal or metals of group VIII and that or those of group IVa being between 0.3 and 3.

2. A process according to claim 1 wherein the catalyst is essentially constituted by the support, at least one metal from group VIII and at least one metal from group IVa.

3. A process according to claim 1 wherein the polyunsaturated cyclic compound is a substituted cyclohexane.

4. A process according to one of claims 1 and 2 wherein the polyunsaturated compound is 4-vinylcyclohexene which is hydrogenated to give vinylcyclohexane.

5. A process according to one of claims 1 to 3 wherein the pressure is between 1 and 5 MPa.

6. A process according to one of claims 1 to 3 wherein the temperature is between 20 and 50°C.

7. A process according to one of the preceding claims wherein the metal of group VIII is rhodium and the metal of group IVa is tin.

8. A process according to one of the preceding claim wherein the catalyst contains from 1 to 3% of at least one metal of group VIII.

9. A process according to one of the preceding claims wherein the molar ratio between the metal (metals) of group VIII and the metal (metals) of group IVa is between 0.8 and 2.5.

## Patentansprüche

1. Verfahren zur selektiven Hydrierung, in Gegenwart eines Katalysators, einer mehrfach ungesättigten cyklischen Verbindung, die wenigstens eine endocyklische Kohlenstoff-Kohlenstoff-Doppelbindung und wenigstens eine endständige Kohlenstoff-Kohlenstoff-Doppelbindung aufweist, wobei die Doppelbindungen nicht konjugiert sind, in einem Gemisch, das im wesentlichen die Verbindung enthält, deren Ring abgesättigt worden ist und die wenigstens eine endständige exocyklische Doppelbindung aufweist,
bei dem die mehrfach ungesättigte Verbindung in Gegenwart von Wasserstoff unter 1 bis 10 MPa und bei einer Temperatur von 1 bis 100°C in Kontakt mit einem Katalysator gebracht wird, der enthält: einen Träger, ausgewählt aus der Gruppe bestehend aus Aluminiumoxid, Siliciumoxid, Silicium-Aluminium-Oxiden und Trägern auf Kohlenstoffgrundlage, und
a)0,1 bis 5 Gew.-% eines Metalls der Gruppe VIII, ausgewählt aus der Gruppe bestehend aus Iridium, Osmium, Nickel, Palladium, Platin, Rhodium, und
b)0,01 bis 15 Gew.-% der Gruppe IVa, ausgewählt aus der Gruppe bestehend aus Zinn, Germanium und Blei, wobei das molare Verhältnis zwischen dem Metall (den Metallen) der Gruppe VIII und dem Metall (den Metallen) der Gruppe IVa zwischen 0,3 und 3 beträgt.

2. Verfahren nach Anspruch 1, bei dem der Katalysator im wesentlichen aus dem Träger, wenigstens einem Metall der Gruppe VIII und einem Metall der Gruppe IVa besteht.

3. Verfahren nach Anspruch 1, bei dem die mehrfach ungesättigte cyklische Verbindung ein substituiertes Cyclohexen ist.

4. Verfahren nach einem der Ansprüche 1 oder 2, bei dem die mehrfach ungesättigte cyklische Verbindung 4-Vinylcyclohexen ist, das zu Vinylcyclohexan substituiert wird.

5. Verfahren nach einem der Ansprüche 1 bis 3, bei dem der Druck zwischen 1 und 5 MPa beträgt.

6. Verfahren nach einem der Ansprüche 1 bis 3, bei dem die Temperatur zwischen 20 und 50°C beträgt.

7. Verfahren nach einem der vorhergehenden Ansprüche, bei dem das Metall der Gruppe VIII Rhodium und das Metall der Gruppe IVa Zinn ist.

8. Verfahren nach einem der vorhergehenden Ansprüche, bei dem der Katalysator 1 bis 3 Gew.-% an wenigstens einem Metall der Gruppe VIII enthält.

9. Verfahren nach einem der vorhergehenden Ansprüche, bei dem das molare Verhältnis zwischen Metall(en) der Gruppe VIII und Metall(en) der Gruppe IVa zwischen 0,8 und 2,5 beträgt.
